# EUROPEAN PATENT SPECIFICATION

(11) **EP 4 512 911 B1**
(45) Date of publication and mention of the grant of the patent: **29.04.2026**
(21) Application number: 23854564.4
(22) Date of filing: 06.11.2023
(51) Int. Cl.: C12Q 1/6895, C12N 15/11, C12Q 1/686, C12C 7/04

(54) **METHOD FOR REGULATING AND CONTROLLING MALT PYF ON BASIS OF GENE EXPRESSION IN MALTING PROCESS AND USE THEREOF**
VERFAHREN ZUR REGULIERUNG UND BEKÄMPFUNG VON MALZ PYF AUF DER BASIS DER GENEXPRESSION IN EINEM MÄLZUNGSVERFAHREN UND VERWENDUNG DAVON
PROCÉDÉ DE RÉGULATION ET DE CONTRÔLE DU PYF DE MALT SUR LA BASE DE L'EXPRESSION GÉNIQUE DANS UN PROCÉDÉ DE MALTAGE ET SON UTILISATION

(30) Priority: 24.11.2022 CN 202211480070
(43) Date of publication of application: 26.02.2025
(73) Proprietor: Tsingtao Brewery Co., Ltd., Shibei District Qingdao, Shandong 266000 (CN)
(72) Inventor: JIANG, Zongxiang, Qingdao, Shandong 266000 (CN); YIN, Hua, Qingdao, Shandong 266000 (CN); YU, Junhong, Qingdao, Shandong 266000 (CN); LIU, Jia, Qingdao, Shandong 266000 (CN); HU, Shumin, Qingdao, Shandong 266000 (CN); HUANG, Shuxia, Qingdao, Shandong 266000 (CN); QIN, Qingqing, Qingdao, Shandong 266000 (CN); HE, Yang, Qingdao, Shandong 266000 (CN); ZHANG, Lei, Qingdao, Shandong 266000 (CN)
(74) Representative: J A Kemp LLP
(86) International application number: PCT/CN2023/129885
(87) International publication number: WO 2024/037669

(56) References cited:
- CN-A- 108 342 458
- CN-A- 108 531 565
- CN-A- 112 143 782
- CN-A- 115 786 576
- CN-A- 116 219 054
- CN-A- 116 413 421
- JP-A- 2008 017 758
- JIBIKI MAKIKO ET AL: "Application of a Newly Developed Method for Estimating the Premature Yeast Flocculation Potential of Malt Samples", vol. 64, no. 2, 1 April 2006 (2006-04-01), US, pages 79 - 85, XP093302971, ISSN: 0361-0470, Retrieved from the Internet <URL:https://www.tandfonline.com/doi/pdf/10.1094/ASBCJ-64-0079> DOI: 10.1094/ASBCJ-64-0079
- APOSTOLOS G PANTELOGLOU ET AL: "Malt-induced premature yeast flocculation: current perspectives", JOURNAL OF INDUSTRIAL MICROBIOLOGY & BIOTECHNOLOGY, SPRINGER, BERLIN, DE, vol. 39, no. 6, 4 February 2012 (2012-02-04), pages 813 - 822, XP035060032, ISSN: 1476-5535, DOI: 10.1007/S10295-012-1086-0
- MANDEEP KAUR ET AL: "TRFLP analysis reveals that fungi rather than bacteria are associated with premature yeast flocculation in brewing", JOURNAL OF INDUSTRIAL MICROBIOLOGY & BIOTECHNOLOGY, SPRINGER, BERLIN, DE, vol. 39, no. 12, 28 August 2012 (2012-08-28), pages 1821 - 1832, XP035140096, ISSN: 1476-5535, DOI: 10.1007/S10295-012-1188-8
- XIE XIN, GUO LIYUN: "Optimization of yeast flocculation gene expression analysis method", GLOBAL ALCINFO., no. 067, 1 May 2018 (2018-05-01), pages 33 - 38, XP093140146
- ZHAO NAN, XIE XIN, GUO LIYUN, HOU HONGXIA: "Study on the effects of differences in malt and yeast varieties on yeast flocculation ", GLOBAL ALCINFO., no. 116, 1 June 2020 (2020-06-01), pages 8 - 12, XP093140149

## Description

The present application claims the priority benefit of Chinese application No. 202211480070.2, filed on November 24, 2022, entitled "Malt PYF Regulation Method Based on Gene Expression during Malting Process and Application Thereof".

### TECHNICAL FIELD

The present application belongs to the field of bioengineering, and particularly relates to a malt PYF regulation method based on gene expression during a malting process and application thereof.

### BACKGROUND

Premature yeast flocculation (PYF) is a phenomenon of yeast flocculating and settling without reaching sufficient fermentation time during the beer fermentation stage. This phenomenon may cause slow degradation of sugar and slow reduction of acetaldehyde during fermentation, resulting in poor maturity of the finished beer, and thus affecting the beer fermentation quality and producing beer quality problems. The PYF problem is a unique quality problem in the beer brewing industry, due to the presence of substances in malt leading to premature yeast flocculation.

At present, for the detection of malt PYF, generally the malt is prepared into wort, then yeast is added for fermentation, and then the number of suspended yeast in the fermentation broth is detected. This detection method needs fermentation time for 40-48 hours; counting the preparation of wort and detection of the yeast, it takes a total of 3-4 days for detection, so the detection time is long. In addition, the method detects the finished malt, which lacks the prediction for the malt production process so cannot adjust the malt production process in time.

JIBIKI MAKIKO ET AL ("Application of a Newly Developed Method for Estimating the Premature Yeast Flocculation Potential of Malt Samples", JOURNAL OF THE AMERICAN SOCIETY OF BREWING CHEMISTS., vol. 64, no. 2, 1 April 2006 (2006-04-01), pages 79-85, XP093302971, US ISSN: 0361-0470, DOI: 10.1094/ASBCJ-64-0079) discloses a fermentation method for estimating the premature yeast flocculation (PYF) potential in malt samples / batches. Controls were prepared from strong PYF-positive malt and PYF-negative malt with normal fermentability and flocculation properties.

### SUMMARY

In order to overcome some of the problems existing in the prior art, the present application provides a malt premature yeast flocculation (PYF) regulation method based on gene expression during a malting process.

The invention is as defined in claim 1.

In an embodiment, the tracking genes are screened by the following method:
conducting transcriptome analysis of the gene expression level differences between the standard malt and the PYF malt in a germination process of the malting process, and selecting multiple genes with larger expression level differences as candidate genes;
tracking the mass production process of the standard malt and the PYF malt, and further selecting 13 candidate genes with the most obvious change trends from the candidate genes as the tracking genes.

In an embodiment, a screening criterion for the tracking genes is that: a gene expression change amplitude is greater than or equal to 2.0 or less than 0.5; wherein the gene expression change amplitude is a ratio of the gene expression level of the PYF malt to the gene expression level of the standard malt.

In an embodiment, the relevant primers designed according to the sequences of the tracking genes include a reverse transcription reaction primer set and a multiplex PCR reaction primer set, used for the reverse transcription reaction to prepare the cDNA template and the multiplex PCR reactions respectively.

In an embodiment, the reverse transcription reaction primer set is designed for the 13 tracking genes AP1, WRKY70, OSM1, GA2ox3, thioredoxin, serpin, RPP13, RbohE, RbohB2, A2, IAA16, BGU17 and WRKY5 to obtain 13 primers, as shown in SEQ ID NO.2, SEQ ID NO.4, SEQ ID NO.6, SEQ ID NO.8, SEQ ID NO.10, SEQ ID NO.12, SEQ ID NO.14, SEQ ID NO.16, SEQ ID NO.18, SEQ ID NO.20, SEQ ID NO.22, SEQ ID NO.24 and SEQ ID NO.26 respectively.

In an embodiment, the multiplex PCR reaction primer set is designed for the 13 tracking genes AP1, WRKY70, OSM1, GA2ox3, thioredoxin, serpin, RPP13, RbohE, RbohB2, A2, IAA16, BGU17 and WRKY5 to obtain 13 primers, as shown in SEQ ID NO.1, SEQ ID NO.3, SEQ ID NO.5, SEQ ID NO.7, SEQ ID NO.9, SEQ ID NO.11, SEQ ID NO.13, SEQ ID NO.15, SEQ ID NO.17, SEQ ID NO.19, SEQ ID NO.21, SEQ ID NO.23 and SEQ ID NO.25 respectively.

In an embodiment, more specifically, the PYF performance of the malt to be tested is predicted by the following prediction criterion:
the principal component scores of the standard malt and the malt to be tested are calculated based on the gene expression levels, and the absolute value of the difference between the scores (ABS) is obtained; wherein,
when the ABS is less than 0.5, the PYF value of the malt to be tested is ≥90%, and the PYF performance of the malt to be tested is predicted to be normal;
when the ABS is more than 0.5 and less than 5.0, the PYF value of the malt to be tested is 50-90%, the PYF performance of the malt to be tested is predicted to be abnormal, and the PYF value needs to be increased;
when the ABS is more than 5.0, the PYF value of the malt to be tested is <50%, the PYF performance of the malt to be tested is predicted to be abnormal, and the PYF value needs to be increased.

In an embodiment, the prediction criterion of the PYF performance of the malt to be tested is obtained by the following method: analyzing gene expression levels of tracking genes of different PYF malts to obtain principal component scores of the different PYF malts; comparing the principal component score of each of the different PYF malts with the principal component score of the standard malt, thereby establishing the prediction criterion.

In an embodiment, the different PYF malts include PYF-90% malt, PYF-50% malt and PYF-30% malt; wherein an absolute value of the difference between a principal component score of the PYF-90% malt and the principal component score of the standard malt is less than 0.5, an absolute value of the difference between a principal component score of the PYF-50% malt and the principal component score of the standard malt is greater than 0.5 and less than 5.0, and an absolute value of the difference between a principal component score of the PYF-30% malt and the principal component score of the standard malt is greater than 5.0.

In an embodiment, when the ABS is greater than 0.5 and less than 5.0 or greater than 5.0, the PYF performance of the malt to be tested is predicted to be abnormal; after adjusting the malting process by reducing the steeping temperature or reducing the fresh air volume and increasing the return air volume during the malting process, the gene expression level is re-analyzed and the principal component score is re-calculated until the ABS is less than 0.5.

The present application also provides an application of the malt PYF regulation method based on gene expression during the malting process described in any of the foregoing embodiments in improving the PYF performance of malt.

Compared with the prior art, the present application has the following advantages and positive effects:
The malt PYF regulation method based on gene expression during the malting process provided by at least one embodiment of the present application has the characteristics of short time consumption, high detection efficiency and high accuracy, etc., which can accurately predict the PYF performance of malt from the malt production process and establish measures for adjusting the malting process based on the prediction results, and ultimately achieve the purpose of improving the PYF performance of malt.

According to the malt PYF regulation method based on gene expression during the malting process provided by at least one embodiment of the present application, by comparing the gene expression level differences between the standard malt and the PYF malts in the malting process and combining the mass production process to select the tracking genes, and then comparing the expression levels of the standard malt and the malt to be tested and combining the evaluation criterion, the accurate prediction of malt PYF performance is achieved; then based on the prediction results, whether the PYF of the malt to be tested is abnormal is judged, if the prediction result is normal, there is no need to adjust the malting process; if the prediction result is abnormal, the measures to reduce the steeping temperature or reduce the fresh air volume and increase the return air volume in the malting process need to be taken to increase the PYF value of the malt, until increased to the expected range.

According to the malt PYF regulation method based on gene expression during the malting process provided by at least one embodiment of the present application, in order to improve the accuracy of the prediction results and further guide the subsequent malt production process more scientifically, a dual criteria including "screening criterion of tracking genes and the evaluation and improvement criterion for malt PYF performance" is established, which can realize the rapid and accurate analysis of malt PYF performance from the malt production process.

### DETAILED DESCRIPTION OF THE EMBODIMENTS

The technical solutions of embodiments in the present application will be described clearly and completely below.

The mass production process known to those skilled in the art refers to the malt production process, i.e. malting process, in the field of beer raw material production.

The PYF value refers to the percentage of the number of suspended yeasts that a malt can meet the requirements of the beer brewing process, which is a judgment value often used by those skilled in the art; it can be used to indicate the difference in yeast suspension performance of wort prepared by mashing different malts during the fermentation process, and it is a relative value. Firstly, a beer manufacturer will select a batch of malt with stable quality and normal yeast suspension performance during the fermentation process as a standard malt, similar to a control malt, then other malts and the standard malt are subjected to EBC (European Brewing Convention) fermentation test to detect the number of suspended yeasts in the fermentation process; and then the number of suspended yeasts of each other malts is divided by the number of suspended yeasts of the standard malt to obtain the PYF value of other malts. For example, if the detected number of suspended yeasts of the standard malt is 10 million/ml and the detected number of suspended yeasts of other malt is 9 million/ml, then the PYF value of the standard malt is 100% and the PYF value of the other malt is 90%, and the other PYF values are also deducted by analogy. Of course, the specific number of suspended yeasts of the standard malt is determined according to the different standard malt selected by each beer manufacturer, and once determined, the PYF value of the standard malt is defined as 100%.

In the present application, the standard malt, PYF malts and malts to be tested are mainly mentioned. As described above, the standard malt refers to malt with a PYF value of 100%; when a standard malt is used for production, there is no need to additionally adjust the steeping temperature of the malting process, or increase or decrease the fresh air volume and return air volume. The PYF malts in the present application mainly includes PYF-90% malt, PYF-50% malt and PYF-30% malt, and can also be malts of other percentages; among them, the PYF-90% malt refers to malt with a PYF value of 90%, indicating that the detected number of suspended yeasts of the malt is 90% of the number of suspended yeasts of the standard malt, which can meet the production requirements, and generally there is no need to additionally adjust the parameters of the malting process; the PYF-50% malt refers to malt with a PYF value of 50%, indicating that the detected number of suspended yeasts of the malt is 50% of the number of suspended yeasts of the standard malt, which cannot meet the production requirements, and generally the PYF value can be increased by reducing the steeping temperature during the malting process; the PYF-30% malt refers to malt with a PYF value of 30%, indicating that the detected number of suspended yeasts of the malt is 30% of the number of suspended yeasts of the standard malt, which cannot meet the production requirements, and generally the PYF value can be increased by reducing the fresh air volume and increasing the return air volume. By comparing the malt to be tested with the standard malt, the PYF value of the malt to be tested might be greater than or equal to 90%, which can meet the production requirements; or the PYF value might be between 50% and 90%, or less than 50%, which cannot meet the production requirements, and the production requirements can be achieved by improving the process; generally, the malt with a PYF value of less than 30% is directly discarded.

In addition, the principal component analysis method mentioned in the present application is a conventional mathematical statistical method. This method is used to analyze and process the obtained gene expression level in some embodiments of the present application to obtain the principal component score of the relevant gene. In the present application, the principal component score of the gene expression level of certain malt can also be referred to as the principal component score of the malt for short.

The first embodiment of the present application provides a malt PYF regulation method based on gene expression during a malting process, including the following steps:
S1, analyzing the gene expression level differences between the standard malt and PYF malts in the malting process, and selecting tracking genes in combination with the mass production process, wherein the tracking genes are AP1, WRKY70, OSM1, GA2ox3, thioredoxin, serpin, RPP13, RbohE, RbohB2, A2, IAA16, BGU17 and WRKY5;
S2, after designing relevant primers according to the sequences of the tracking genes, in sequence, extracting total RNA, preparing cDNA template by reverse transcription reaction, performing multiplex PCR reaction, performing electrophoresis and performing gene expression level analysis of product fragments for a malt to be tested and the standard malt respectively; and predicting and improving the PYF performance of the malt to be tested according to the following criteria:
   calculating the principal component scores of the standard malt and the malt to be tested based on the gene expression levels, and taking the absolute value of the difference between the two (abbreviated as ABS); wherein,
   when the ABS is less than 0.5, the PYF value of the malt to be tested is ≥90%, the PYF performance of the malt to be tested is predicted to be normal, and there is no need to adjust the malting process;
   when the ABS is more than 0.5 and less than 5.0 or more than 5.0, the PYF performance of the malt to be tested is predicted to be abnormal, and the steeping temperature needs to be reduced or the fresh air volume needs to be reduced and the return air volume needs to be increased during the malting process.

The functions of the 13 tracking genes mentioned in the above embodiment are as follows:

| Gene | Function |
|---|---|
| AP1 | Acid phosphatase |
| WRKY70 | WRKY DNA-binding protein 54 |
| OSM1 | Pathogenesis-related thaumatinsuperfamily protein |
| GA2ox3 | Decomposition of GA in cells |
| thioredoxin | Thioredoxin |
| serpin | Enzyme inhibitory protein-serpin |
| RPP13 | Disease resistance protein RPP 13 |
| RbohE | Production of intracellular ROS |
| RbohB2 | Production of intracellular ROS |
| A2 | Peroxidase superfamily protein |
| IAA16 | Auxin-responsive protein IAA16 |
| BGU17 | beta glucosidase 17 |
| WRKY5 | WRKY DNA-binding protein 50 |

Further, in the present embodiment, for the malt to be tested with abnormal PYF performance prediction, the reason for reducing the steeping temperature of the malting process or reducing the fresh air volume and increasing the return air volume is that the measure can inhibit the growth of microorganisms and reduce the germination rate of barley, thereby lowering the possibility of microbial contamination and reducing the production of PYF factors.

The tracking genes can be screened by the following method:
conducting transcriptome analysis of the gene expression level difference between the standard malt and the PYF malts in the germination process during the malting process, and selecting multiple genes with larger expression level differences as candidate genes; obviously, the number of candidate genes is more than the number of tracking genes, for example, about 20 genes with the gene expression change amplitude of greater than 1.5 or less than 0.66 are selected.
tracking the mass production process of the standard malt and the PYF malts, and further selecting 13 candidate genes with the most obvious change trends from the candidate genes as the tracking genes.

In a specific embodiment, the screening criterion for the tracking genes is that: the gene expression change amplitude is greater than or equal to 2.0 or less than 0.5; wherein the gene expression change amplitude is the ratio of the gene expression level of the PYF malt to the gene expression level of the standard malt. The purpose of setting the screening criterion for tracking genes at a gene expression change amplitude of greater than or equal to 2.0 or less than 0.5 is mainly to find genes with the most obvious expression level differences before and after as the characteristic genes (i.e., tracking genes). It is generally believed that an expression level difference of more than 2 times is considered to be an obvious difference. It can be increased to more than twofold or reduced to less than 1/2 of the original level, so the gene expression change amplitude of greater than or equal to 2.0 or less than 0.5 is used as the criterion.

In a specific embodiment, in the step S2, the relevant primers designed according to the tracking gene sequence include a reverse transcription reaction primer set and a multiplex PCR reaction primer set, used for the reverse transcription reaction to prepare the cDNA template and the multiplex PCR reaction, respectively.

The reverse transcription reaction primer set is designed for the 13 tracking genes AP1, WRKY70, OSM1, GA2ox3, thioredoxin, serpin, RPP13, RbohE, RbohB2, A2, IAA16, BGU17 and WRKY5 to obtain 13 primers, as shown in SEQ ID NO.2, SEQ ID NO.4, SEQ ID NO.6, SEQ ID NO.8, SEQ ID NO.10, SEQ ID NO.12, SEQ ID NO.14, SEQ ID NO.16, SEQ ID NO.18, SEQ ID NO.20, SEQ ID NO.22, SEQ ID NO.24 and SEQ ID NO.26 respectively.

The multiplex PCR reaction primer set is designed for the 13 tracking genes AP1, WRKY70, OSM1, GA2ox3, thioredoxin, serpin, RPP13, RbohE, RbohB2, A2, IAA16, BGU17 and WRKY5 to obtain 13 primers, as shown in SEQ ID NO.1, SEQ ID NO.3, SEQ ID NO.5, SEQ ID NO.7, SEQ ID NO.9, SEQ ID NO.11, SEQ ID NO.13, SEQ ID NO.15, SEQ ID NO.17, SEQ ID NO.19, SEQ ID NO.21, SEQ ID NO.23 and SEQ ID NO.25 respectively.

In a specific embodiment, the PYF performance of the malt to be tested can be more specifically predicted using the following prediction criterion:
calculating the principal component scores of the standard malt and the malt to be tested based on the gene expression levels, and taking the absolute value ABS of the difference between the two; wherein,
when the ABS is less than 0.5, the PYF value of the malt to be tested is ≥90%, and the PYF performance of the malt to be tested is predicted to be normal;
when the ABS is more than 0.5 and less than 5.0, the PYF value of the malt to be tested is 50-90%, the PYF performance of the malt to be tested is predicted to be abnormal, and the PYF value needs to be improved;
when the ABS is more than 5.0, the PYF value of the malt to be tested is <50%, the PYF performance of the malt to be tested is predicted to be abnormal, and the PYF value needs to be improved.

In a specific embodiment, the prediction criterion of the PYF performance of the malt to be tested is obtained by the following method: (1) performing gene expression level analysis of the tracking genes of different PYF malts to obtain the principal component scores of the gene expression levels; (2) comparing the principal component scores of the gene expression levels of the different PYF malts with the principal component score of the gene expression level of the standard malt, thereby establishing the prediction criterion of the PYF performance of the malt to be tested.

The different PYF malts mentioned in the above step (1) refer to malts with different PYF values, which specifically include PYF-90% malt, PYF-50% malt and PYF-30% malt in the present embodiment. By analyzing the gene expression of malts with different PYF values, the obtained principal component scores and the prediction criterion obtained based on the scores are more accurate.

In the embodiment, the reason for selecting the principal component score of the gene expression level as the analysis index is as follows: in order to comprehensively evaluate multiple gene expression levels, the principal component analysis method is used to perform dimension reduction process of the multiple gene expression levels (gene expression data), so as to obtain the comprehensive quantitative scores of the multiple genes. In an embodiment, when the ABS is more than 0.5 and less than 5.0 or more than 5.0, the PYF performance of the malt to be tested is predicted to be abnormal; after adjusting the malting process by reducing the steeping temperature or reducing the fresh air volume and increasing the return air volume during the malting process, the gene expression level analysis is re-performed and the principal component score is re-calculated until the ABS is less than 0.5. More specifically, when the ABS is more than 0.5 and less than 5.0, the PYF performance of the malt to be tested is predicted to be abnormal; after adjusting the malting process by reducing the steeping temperature during the malting process, the gene expression level analysis and the principal component score calculation are re-performed until the ABS is less than 0.5. When the ABS is more than 5.0, the PYF performance of the malt to be tested is predicted to be abnormal; after adjusting the malting process by reducing the fresh air volume and increasing the return air volume, the gene expression level analysis and the principal component score calculation are re-performed until the ABS is less than 0.5.

The second embodiment of the present application provides an application of the malt PYF regulation method based on gene expression during the malting process as described in any of the foregoing embodiments in improving the PYF performance of the malt to be tested.

Furthermore, in order to describe more clearly about the malt PYF regulation method based on gene expression during the malting process and the application thereof provided by the embodiments of the present application, it will be described in conjunction with specific examples as below.

### Example 1

The present example provided a screening process of candidate genes and tracking genes in a malt PYF performance prediction method based on barley gene expression during the malting process.

Studies showed that, the produced malt PYF problems are related to the stress response caused by external stimulation of barley during the planting and germination process. Therefore, in the present example, through transcriptome analysis of gene expression level differences between standard malt and PYF malts during the malting process, especially during the seed germination, multiple genes with great expression level differences (i.e., candidate genes) were found, and 13 genes with the most obvious differences were selected as monitoring genes (i.e., tracking genes);
More specifically, after candidate genes (about 20 genes with gene expression change amplitude of more than 1.5 or less than 0.66) were obtained, by tracking the mass production process (i.e., in the malting process, green malt was taken after steeping; in order to ensure that the life activity of the green malt did not change, the sample that was taken out should be subjected to RNA extraction immediately or placed at -80°C for storage), 13 genes with the most obvious change trend were selected as tracking genes. The screening criterion for tracking genes was that the gene expression change amplitude was more than or equal to 2.0 or less than 0.5; the gene expression change amplitude was the ratio of the gene expression level of PYF malt to the gene expression level of standard malt. Table 1 showed the gene expression levels of the standard malt and some PYF malts. The gene expression level of malt can be obtained by the prior art, which will be further described in step (6) of the subsequent Example 3.

**Table 1 Statistics of gene expression levels in standard malt and PYF malt**

| No. | Gene | Gene expression level in standard malt X1 | Gene expression level in PYF malt X2 | Gene expression change amplitude X2/X1 |
|---|---|---|---|---|
| 1 | AP1 | 0.39 | 5.81 | 15.0 |
| 2 | WRKY70 | 0.66 | 5.19 | 7.9 |
| 3 | OSM1 | 0.48 | 2.94 | 6.1 |
| 4 | GA2ox3 | 0.92 | 4.32 | 4.7 |
| 5 | thioredoxin | 0.40 | 1.68 | 4.2 |
| 6 | serpin | 0.38 | 1.50 | 4.0 |
| 7 | RPP13 | 0.79 | 2.07 | 2.6 |
| 8 | RbohE | 0.73 | 1.71 | 2.3 |
| 9 | RbohB2 | 0.55 | 1.26 | 2.3 |
| 10 | A2 | 0.86 | 1.72 | 2.0 |
| 11 | IAA16 | 1.10 | 0.48 | 0.4 |
| 12 | BGU17 | 2.30 | 0.97 | 0.4 |
| 13 | WRKY5 | 9.19 | 1.53 | 0.2 |

### Example 2

The present example provided a determination process of the malt PYF performance prediction criterion in the malt PYF performance prediction method based on barley gene expression during the malting process, specifically:
The gene expression of three different PYF malts (i.e., PYF-90% malt, PYF-50% malt, and PYF-30% malt) were analyzed and detected respectively, to obtain the gene expression levels, and then the principal component scores of the gene expression levels were obtained by the principal component analysis method; and the relevant criterion was determined by comparing with the score of the standard malt. Table 2 showed the gene expression levels of the standard malt and the three PYF malts, as well as the principal component scores obtained by the principal component analysis of these gene expression levels.

**Table 2 Principal component scores of the standard malt and the three PYF malts**

| No. | Gene | Gene expression level of standard malt | Gene expression level of PYF-90% malt | Gene expression level of PYF-50% malt | Gene expression level of PYF-30% malt |
|---|---|---|---|---|---|
| 1 | AP1 | 0.39 | 0.35 | 0.31 | 0.98 |
| 2 | WRKY70 | 0.66 | 0.62 | 0.69 | 1.35 |
| 3 | OSM1 | 0.48 | 0.28 | 1.25 | 1.45 |
| 4 | GA2ox3 | 0.92 | 0.54 | 0.37 | 0.21 |
| 5 | thioredoxin | 0.40 | 0.40 | 1.22 | 0.99 |
| 6 | serpin | 0.38 | 0.32 | 0.95 | 1.35 |
| 7 | RPP13 | 0.79 | 0.71 | 0.37 | 1.48 |
| 8 | RbohE | 0.73 | 0.69 | 0.48 | 1.58 |
| 9 | RbohB2 | 0.55 | 0.55 | 1.05 | 0.15 |
| 10 | A2 | 0.86 | 0.8 | 0.68 | 0.42 |
| 11 | IAA16 | 1.10 | 1.01 | 1.21 | 0.82 |
| 12 | BGU17 | 2.30 | 2.20 | 1.92 | 1.05 |
| 13 | WRKY5 | 9.19 | 9.23 | 10.23 | 4.35 |
| Principal component scores | | -1.335 | -1.055 | 0.325 | 4.733 |
| ABS | | | 0.280 | 1.660 | 6.068 |

According to the data in the above table, by comparing the principal component scores of the gene expression levels of the malts with different PYF values with the standard malt respectively, it was found that, the absolute value of the difference between the principal component score of the gene expression level of the PYF-90% malt and the principal component score of the standard malt was less than 0.5, the absolute value of the difference between the principal component score of the gene expression level of the PYF-50% malt and the principal component score of the standard malt was more than 0.5 and less than 5, and the absolute value of the difference between the principal component score of the gene expression level of the PYF-30% malt and the principal component score of the standard malt was greater than 5.

Therefore, the prediction criterion for the malt to be tested was determined as follows: When the absolute value (ABS) of the difference between the principal component scores of the malt to be tested and the standard malt is less than 0.5, it is inferred that the PYF value of the malt to be tested is more than 90%, and the PYF performance is predicted to be normal. When the ABS is more than 0.5 and less than 5.0, it is inferred that the PYF value of the malt to be tested is between 50% and 90%, the PYF performance is predicted to be abnormal, and the PYF value needs to be improved. When the ABS is more than 5.0, it is inferred that the PYF value of the malt to be tested is less than 50%, the PYF performance is predicted to be abnormal, and the PYF value needs to be improved.

### Example 3

This example provided a malt PYF regulation method based on gene expression during the malting process. In the present example, the PYF performance of the purchased batch A barley was predicted and improved, specifically:
(1) Selection of tracking genes: 13 genes including AP1, WRKY70, OSM1, GA2ox3, thioredoxin, serpin, RPP13, RbohE, RbohB2, A2, IAA16, BGU17 and WRKY5 were selected as tracking genes.
(2) Sampling: During the malting process, green malt after steeping was taken as the malt to be tested (note: in order to ensure that the life activity of the green malt did not change, the sample that was taken should be subjected to RNA extraction immediately or placed at -80°C for storage).
(3) Crushing of green malt: In order to ensure that the RNA in the green malt was not degraded, the green malt was ground and crushed by using liquid nitrogen.
(4) Extraction of total RNA:
   After the green malt was crushed, total RNA was extracted by adding Trizol (note: Trizol was a total RNA extraction reagent, which could be used to extract total RNA from cells or tissues).

Specifically, the procedure was as follows: take 50-100 mg of sample (the crushed green malt), add 1 ml of Trizol, shake and lyse, then centrifuge at 4°C and 12,000 g for 5 min, transfer the supernatant to a Phasemarker tube and allow to stand for 5 min; add 0.2 ml of chloroform, shake manually for 15s, allow to stand for 10 min; centrifuge at 4°C and 12,000 g for 10 min; transfer the supernatant (450-550 µl) to an EP tube, add 250 µl of 96% ethanol, and mix with a pipette tip; transfer the mixed solution to a silicone tube, centrifuge at 12,000 g for 1 min, and remove the waste liquid; add 700 µl of WB1, centrifuge at 12,000 g for 1 min, and remove the waste liquid. Add 500 µl of WB2, centrifuge at 12,000 g for 1 min, and remove the waste liquid; add 500 µl of WB2, centrifuge at 12,000 g for 1 min, move the silicone tube to the 1.5 ml EP tube; add 50 µl of nuclease-free water, allow to stand for 1 min, and centrifuge at 12,000 g for 1 min; collect the RNA solution after centrifugation to obtain the purified total RNA of barley cells, and might store at -80°C; the WB1 and WB2 are both eluents, and ThermoFisher's PureLink^{™} RNA Mini-Extraction Kit (including WB1 and WB2) (Article No.: 12183018A) can be used.

### (5) Reverse transcription-polymerase chain reaction (RT-PCR):

Preparation of cDNA template by reverse transcription reaction: with the purified total RNA of barley cells obtained in the step (4) as a template, adopting GenomeLabTMGeXP Starter Kit from Beckman Coulter Company, using the downstream primers of the designed multiplex primers as specific primers (with the sequence shown in Table 3), a cDNA first chain (as a cDNA template) was synthesized with the total RNA of barley cells as the template, the reaction system was 10 µL, wherein the synthesis reaction parameters of the cDNA first chain could be set as follows: 48°C for 1 min; 42°C for 60 min; 95°C for 5 min.

Multiplex PCR reaction: the DNA polymerase and GenomeLabTMGeXP Starter Kit from Beckman Coulter Company were adopted. Using the above synthesized cDNA first chain as a template and the upstream primers of the above 13 multiplex primers as specific primers (with the sequences shown in Table 3), the RT-PCR amplification reaction was performed. The RT-PCR amplification parameters could be set as follows: predenaturation at 95°C for 10 min; denaturation at 94°C for 30 s; annealing at 56°C for 30 s; extension at 71°C for 1 min, a total of 35 cycles; finally the reverse transcription amplification product of the expressed gene was obtained.

**Table 3: Sequences of upstream and downstream primers**

| Gene | Upstream and downstream primers (5'->3') | |
|---|---|---|
| AP1 | Forward Primer (Upstream primer) | SEQ ID NO.1 |
| | Reverse primer (Downstream primer) | SEQ ID NO.2 |
| WRKY70 | Upstream primer | SEQ ID NO.3 |
| | Downstream primer | SEQ ID NO.4 |
| OSM1 | Upstream primer | SEQ ID NO.5 |
| | Downstream primer | SEQ ID NO.6 |
| GA2ox3 | Upstream primer | SEQ ID NO.7 |
| | Downstream primer | SEQ ID NO.8 |
| thioredoxin | Upstream primer | SEQ ID NO.9 |
| | Downstream primer | SEQ ID NO.10 |
| serpin | Upstream primer | SEQ ID NO.11 |
| | Downstream primer | SEQ ID NO.12 |
| RPP13 | Upstream primer | SEQ ID NO.13 |
| | Downstream primer | SEQ ID NO.14 |
| RbohE | Upstream primer | SEQ ID NO.15 |
| | Downstream primer | SEQ ID NO.16 |
| RbohB2 | Upstream primer | SEQ ID NO.17 |
| | Downstream primer | SEQ ID NO.18 |
| A2 | Upstream primer | SEQ ID NO.19 |
| | Downstream primer | SEQ ID NO.20 |
| IAA16 | Upstream primer | SEQ ID NO.21 |
| | Downstream primer | SEQ ID NO.22 |
| BGU17 | Upstream primer | SEQ ID NO.23 |
| | Downstream primer | SEQ ID NO.24 |
| WRKY5 | Upstream primer | SEQ ID NO.25 |
| | Downstream primer | SEQ ID NO.26 |

### (6) Analysis of gene expression levels:

The above reverse transcription amplification products were quantitatively analyzed by capillary electrophoresis. Specifically, 1 µl of multiplex PCR product (i.e., the reverse transcription amplification product) was added to the wells of the sample loading plate containing 39 µl of 95% deionized formamide (SLS) and 400 bp Marker mixed solution, mixed with a pipette and covered with a drop of paraffin oil;
Furthermore, 250 µl of separation buffer was added to each well of the sample loading plate. After the preparatory work was completed, capillary electrophoresis was performed on the machine. After completion of the electrophoresis, the gene expression levels of different genes (13 tracking genes) of the malt to be tested were obtained, as shown in Table 4.

In addition, by using the above steps (1) to (6), the gene expression levels of the standard malt, PYF-90% malt, PYF-50% malt and PYF-30% malt could be obtained respectively; the gene expression level of the standard malt was also listed in Table 4. Among them, many of the processing methods for gene editing mentioned in the steps (4), (5) and (6) were conventional methods. At least the upstream primers and downstream primers used in the example were different.

It should be understood that the gene expression level could be obtained by the prior art, and it is actually a value relative to the internal reference gene, which was well known to those skilled in the art. For example, in the present embodiment, for the analysis of gene expression level, the plant activator protein Actin could be used as an internal reference gene act, and the reverse transcription and capillary electrophoresis were performed by using the GeXP equipment from Beckman Coulter company to obtain the electrophoresis peak areas of the malt to be tested and the internal reference gene act respectively; the ratio of peak areas was the gene expression level of the malt to be tested. Using the same internal reference gene act and the same GeXP equipment, the gene expression levels of the standard malt, PYF-90% malt, PYF-50% malt and PYF-30% malt could also be obtained respectively.

### (7) Prediction of PYF performance:

The principal component analysis was performed on the gene expression levels of the batch A malt and the standard malt (data in Table 4) to obtain the corresponding principal component scores.

**Table 4 Gene expression levels of the batch A malt and the standard malt**

| No. | Gene | Gene expression level of standard malt | Gene expression level of batch A malt |
|---|---|---|---|
| 1 | AP1 | 0.39 | 0.33 |
| 2 | WRKY70 | 0.66 | 0.58 |
| 3 | OSM1 | 0.48 | 0.34 |
| 4 | GA2ox3 | 0.92 | 0.67 |
| 5 | thioredoxin | 0.4 | 0.51 |
| 6 | serpin | 0.38 | 0.42 |
| 7 | RPP13 | 0.79 | 0.68 |
| 8 | RbohE | 0.73 | 0.78 |
| 9 | RbohB2 | 0.55 | 0.66 |
| 10 | A2 | 0.86 | 1.02 |
| 11 | IAA16 | 1.1 | 0.98 |
| 12 | BGU17 | 2.3 | 2.18 |
| 13 | WRKY5 | 9.19 | 10.01 |

Through principal component analysis, the principal component score of the standard malt was -1.563 and the principal component score of the batch A malt was -1.628, with an absolute value of the difference between them of 0.065, which was less than 0.5. According to the prediction criterion in Example 2, it was inferred that the PYF value of batch A malt was more than 90%. In addition, the conventional evaluation method was used to measure the fermentation of batch A malt, and the obtained PYF value was 99% (as shown in the subsequent Example 6 for detail). It is indicated that the prediction obtained by this method was reliable, and both methods could be used to judge that the malt PYF performance was predicted to be normal and there was no need to adjust the process. Furthermore, the prediction result obtained by the method provided in the present embodiment was reliable and was obtained more quickly.

### Example 4

This example provided a malt PYF regulation method based on gene expression during the malting process. In the present example, the PYF performance of the purchased batch B barley was predicted and improved.

The specific prediction method was the same as that in Example 3, except that the barley batch was different. The gene expression level of batch B malt could be obtained by the method in Example 3. The standard malt needed not be measured again while the gene expression level measured in Example 3 could be used. The specific data was shown in Table 5.

The principal component analysis was performed on the gene expression levels of the batch B malt and the standard malt to obtain the corresponding principal component scores.

**Table 5 Gene expression levels of the batch B malt and the standard malt**

| No. | Gene | Gene expression level of standard malt | Gene expression level of batch B malt -before adjusting | Gene expression level of batch B malt - after adjusting |
|---|---|---|---|---|
| 1 | AP1 | 0.39 | 0.83 | 0.34 |
| 2 | WRKY70 | 0.66 | 1.28 | 0.57 |
| 3 | OSM1 | 0.48 | 0.89 | 0.41 |
| 4 | GA2ox3 | 0.92 | 0.31 | 0.87 |
| 5 | thioredoxin | 0.40 | 0.92 | 0.52 |
| 6 | serpin | 0.38 | 1.42 | 0.76 |
| 7 | RPP13 | 0.79 | 0.36 | 0.72 |
| 8 | RbohE | 0.73 | 0.29 | 0.68 |
| 9 | RbohB2 | 0.55 | 1.49 | 0.72 |
| 10 | A2 | 0.86 | 1.71 | 0.79 |
| 11 | IAA16 | 1.10 | 0.82 | 1.32 |
| 12 | BGU17 | 2.30 | 1.35 | 1.87 |
| 13 | WRKY5 | 9.19 | 13.52 | 10.1 |

Based on the data in the above table and through the principal component analysis, the principal component score of the standard malt was -1.385, and the principal component score of the batch B malt was 0.181, with an absolute value of the difference between them of 1.566, which was more than 0.5 and less than 5. According to the prediction criterion in Example 2, it was inferred that the PYF value of batch B malt was between 50% and 90%. In addition, the conventional evaluation method was used to measure the fermentation of batch B malt, and the obtained PYF value was 78% (as shown in the subsequent Example 6 for detail). The PYF value was low judged by the two methods, so the PYF performance was predicted to be abnormal, and the process needed to be adjusted to increase the PYF value. Accordingly, the prediction result obtained by the method provided in the present embodiment was reliable and was obtained more quickly.

In the present example, in order to improve the PYF value, the steeping temperature was reduced from 18°C to 16°C during the malting process, and the gene expression level was re-analyzed, then the principal component score was calculated, which was -1.393; the absolute value of the difference between them was 0.008, which was less than 0.5. It was inferred that the improved PYF value was more than 90%. In addition, the PYF value of the batch B malt was 91% obtained by the conventional evaluation method with longer time, which could meet the production requirements. (Note: Studies have shown that the PYF problem was related to influence of external factors on barley during the malting process, such as microbial contamination. Lowering the steeping temperature could reduce the growth rate of contaminant microorganisms and the germination rate of barley, which was conducive to alleviating the PYF problem and improving the PYF value.)

### Example 5

This example provided a malt PYF regulation method based on gene expression during the malting process. In the present example, the PYF performance of the purchased batch C barley was predicted and improved.

The specific prediction method was the same as that in Example 3, except that the barley batch was different. The gene expression level of batch C malt could be obtained by the method in Example 3. For the standard malt, the gene expression level measured in Example 3 could be used. The specific data was shown in Table 6.

The principal component analysis was performed on the gene expression levels of the batch C malt and the standard malt to obtain the corresponding principal component scores.

**Table 6 Gene expression levels of the batch C malt and the standard malt**

| No. | Gene | Gene expression level of standard malt | Gene expression level of batch C malt -before adjusting | Gene expression level of batch C malt - after adjusting |
|---|---|---|---|---|
| 1 | AP1 | 0.39 | 1.43 | 0.42 |
| 2 | WRKY70 | 0.66 | 1.28 | 1.01 |
| 3 | OSM1 | 0.48 | 1.75 | 1.1 |
| 4 | GA2ox3 | 0.92 | 0.21 | 0.89 |
| 5 | thioredoxin | 0.40 | 1.39 | 0.82 |
| 6 | serpin | 0.38 | 1.52 | 0.63 |
| 7 | RPP13 | 0.79 | 1.56 | 0.72 |
| 8 | RbohE | 0.73 | 1.89 | 0.81 |
| 9 | RbohB2 | 0.55 | 1.78 | 0.65 |
| 10 | A2 | 0.86 | 0.22 | 0.22 |
| 11 | IAA16 | 1.10 | 0.51 | 0.51 |
| 12 | BGU17 | 2.30 | 0.55 | 0.55 |
| 13 | WRKY5 | 9.19 | 3.53 | 3.53 |

Based on the data in the above table and through the principal component analysis, the principal component score of the standard malt was -1.385, and the principal component score of the batch C malt was 4.241, with an absolute value of the difference between them of 5.627, which was more than 5.0. According to the prediction criterion in Example 2, it was inferred that the PYF value of batch C malt was less than 50%. In addition, the conventional evaluation method was used to measure the fermentation of batch C malt, and the obtained PYF value was 43% (as shown in the subsequent Example 6 for detail). The PYF value of this batch of malt was low judged by the two methods, so the PYF performance was predicted to be abnormal, and the process needed to be adjusted to increase the PYF value.

In the present example, in order to improve the PYF value, the fresh air volume was reduced from 100% to 50% and the return air volume was increased to 50%, and the gene expression level was re-analyzed, then the principal component score was calculated, which was -0.359; the absolute value of the difference between them was 1.026, which was more than 0.5 and less than 5.0. It was inferred that the improved PYF value was in the range of 50-90%. In addition, the PYF value of the batch C malt was increased to 82% obtained by the conventional evaluation method with longer time. (Note: Studies have shown that the PYF problem was related to influence of external factors on barley during the malting process, such as microbial contamination. Reducing the fresh air volume and increasing the return air volume could reduce the growth of contaminant microorganisms and the growth of barley, which was conducive to alleviating the PYF problem and improving the PYF value.)

### Example 6: Verification test

In order to further verify the accuracy of the malt PYF regulation method based on gene expression during the malting process, the malt PYF performance prediction method was verified (verification test I), and further the malt PYF performance improvement method was verified (verification test II), specifically as follows:
1. Verification test I: The conventional evaluation method was used for result verification, specifically as follows:
   The conventional evaluation method was performed (i.e., the malt was prepared into wort, then the yeast was added for fermentation, and the number of suspended yeasts in the fermentation broth was detected), and the results were compared with the results of Examples 3 to 5.

The conventional evaluation method was specifically as follows:
(1) preparation of wort: a certain amount of malt was crushed and then saccharified with water, and then filtered to obtain saccharified wort;
(2) inoculated fermentation of yeasts: a certain amount of yeast paste was placed in the sterilized wort, shaken and oxygenated, and then fermented at 20°C for 48 h;
(3) counting of yeasts: a certain amount of fermentation broth was taken and the yeasts were counted by using a yeast counter, and the yeast concentration in the fermentation broth was obtained;
(4) calculation of PYF value: PYF value = number of yeast cells in the sample/ number of yeast cells in the standard sample × 100%.

**Table 7 Comparison of results obtained by the conventional evaluation method with the results in Examples 3 to 5**

| No. | PYF value | |
|---|---|---|
| | Conventional evaluation method | Prediction methods in the application |
| Example 3 | 99% | > 90% |
| Example 4 | 78% | 50%-90% |
| Example 5 | 43% | Less than 50% |

As shown in Table 7, compared with the conventional evaluation method, the method of the present application compared the gene expression level differences between the standard malt and the PYF malt during the malting process, and selected the tracking genes in combination with the mass production process, and then compared the gene expression levels and the principal component scores of the standard malt and the malt to be tested, to achieve the accurate prediction of PYF performance of the malt to be tested.

### 2. Verification Test II

The present verification test was conducted on the batch B malt and batch C malt after adjustment of the malting process in Examples 4 and 5. The results were verified by the conventional evaluation method. The specific procedures were the same as those of the verification test I, and the only difference was that the malt used was the batch B malt and batch C after process adjustment. The test results were as follows:

**Table 8 Comparison of results obtained by the conventional evaluation method with the results in Examples 4 and 5**

| No. | PYF value | |
|---|---|---|
| | Conventional evaluation method | Improvement methods in the application |
| Example 4- batch B malt after adjusting | 91% | >90% |
| Example 5- batch C malt after adjusting | 82% | 50-90% |

After comprehensive analysis of the test results after improvement in Table 8 and the results before improvement in Table 7, it is known that the improvement methods provided in Examples 4 and 5 of the present application could be used to establish adjustment measures for the malting process based on the prediction results and ultimately achieve the purpose of improving the malt PYF performance.

## Claims

1. A malt premature yeast flocculation (PYF) regulation method based on gene expression during a malting process, **characterised in that**, including the following steps:
analyzing gene expression level differences between standard malt and PYF malt in the malting process, and selecting tracking genes in combination with mass production process, wherein the tracking genes are AP1, WRKY70, OSM1, GA2ox3, thioredoxin, serpin, RPP13, RbohE, RbohB2, A2, IAA16, BGU17 and WRKY5;
after designing relevant primers according to sequences of the tracking genes, then in sequence, extracting total RNA, preparing cDNA template by reverse transcription reaction, performing multiplex PCR reaction for said 13 tracking genes, performing electrophoresis and analyzing gene expression level of product fragments for malt to be tested and the standard malt respectively; and predicting and improving PYF performance of the malt to be tested according to the following criteria:
calculating principal component scores of the standard malt and the malt to be tested based on the gene expression levels of said 13 tracking genes, to obtain an absolute value of the difference between the scores (ABS); wherein,
when the ABS is less than 0.5, a PYF value of the malt to be tested is ≥90%, the PYF performance of the malt to be tested is predicted to be normal, and there is no need to adjust the malting process;
when the ABS is more than 0.5 and less than 5.0 or more than 5.0, the PYF performance of the malt to be tested is predicted to be abnormal, and steeping temperature needs to be reduced or fresh air volume needs to be reduced and return air volume needs to be increased during the malting process,
wherein the standard malt refers to a malt with a PYF value of 100%.

2. The malt PYF regulation method according to claim 1, wherein, the tracking genes are screened by the following method:
conducting transcriptome analysis of the gene expression level differences between the standard malt and the PYF malt in a germination process of the malting process, and selecting multiple genes with larger expression level differences as candidate genes;
tracking the mass production process of the standard malt and the PYF malt, and further selecting 13 candidate genes with the most obvious change trends from the candidate genes as the tracking genes.

3. The malt PYF regulation method according to claim 2, wherein, a screening criterion for the tracking genes is that: a gene expression change amplitude is greater than or equal to 2.0 or less than 0.5; wherein the gene expression change amplitude is a ratio of a gene expression level of the PYF malt to the gene expression level of the standard malt.

4. The malt PYF regulation method according to any one of claims 1-3, wherein, the relevant primers designed according to the sequences of the tracking genes include a reverse transcription reaction primer set and a multiplex PCR reaction primer set, used for the reverse transcription reaction to prepare the cDNA template and the multiplex PCR reactions respectively.

5. The malt PYF regulation method according to claim 4, wherein, the reverse transcription reaction primer set is designed for the 13 tracking genes AP1, WRKY70, OSM1, GA2ox3, thioredoxin, serpin, RPP13, RbohE, RbohB2, A2, IAA16, BGU17 and WRKY5 to obtain 13 primers, as shown in SEQ ID NO.2, SEQ ID NO.4, SEQ ID NO.6, SEQ ID NO.8, SEQ ID NO.10, SEQ ID NO.12, SEQ ID NO.14, SEQ ID NO.16, SEQ ID NO.18, SEQ ID NO.20, SEQ ID NO.22, SEQ ID NO.24 and SEQ ID NO.26 respectively.

6. The malt PYF regulation method according to claim 4, wherein, the multiplex PCR reaction primer set is designed for the 13 tracking genes AP1, WRKY70, OSM1, GA2ox3, thioredoxin, serpin, RPP13, RbohE, RbohB2, A2, IAA16, BGU17 and WRKY5 to obtain 13 primers, as shown in SEQ ID NO.1, SEQ ID NO.3, SEQ ID NO.5, SEQ ID NO.7, SEQ ID NO.9, SEQ ID NO.11, SEQ ID NO.13, SEQ ID NO.15, SEQ ID NO.17, SEQ ID NO.19, SEQ ID NO.21, SEQ ID NO.23 and SEQ ID NO.25 respectively.

7. The malt PYF regulation method according to any one of claims 1-3, 5 and 6, wherein, the PYF performance of the malt to be tested is predicted by the following prediction criterion:
the principal component scores of the standard malt and the malt to be tested are calculated based on the gene expression levels, and the absolute value ABS of the difference between the scores is obtained; wherein,
when the ABS is less than 0.5, the PYF value of the malt to be tested is ≥90%, and the PYF performance of the malt to be tested is predicted to be normal;
when the ABS is more than 0.5 and less than 5.0, the PYF value of the malt to be tested is 50-90%, the PYF performance of the malt to be tested is predicted to be abnormal, and the PYF value needs to be increased;
when the ABS is more than 5.0, the PYF value of the malt to be tested is <50%, the PYF performance of the malt to be tested is predicted to be abnormal, and the PYF value needs to be increased.

8. The malt PYF regulation method according to claim 7, wherein, the prediction criterion of the PYF performance of the malt to be tested is obtained by the following method: analyzing gene expression levels of tracking genes of different PYF malts to obtain principal component scores of the different PYF malts; comparing the principal component score of each of the different PYF malts with the principal component score of the standard malt, thereby establishing the prediction criterion.

9. The malt PYF regulation method according to claim 8, wherein, the different PYF malts include PYF-90% malt, PYF-50% malt and PYF-30% malt; wherein an absolute value of the difference between a principal component score of the PYF-90% malt and the principal component score of the standard malt is less than 0.5, an absolute value of the difference between a principal component score of the PYF-50% malt and the principal component score of the standard malt is greater than 0.5 and less than 5.0, and an absolute value of the difference between a principal component score of the PYF-30% malt and the principal component score of the standard malt is greater than 5.0.

10. The malt PYF regulation method according to any one of claims 1-3, 5 and 6, wherein, when the ABS is greater than 0.5 and less than 5.0 or greater than 5.0, the PYF performance of the malt to be tested is predicted to be abnormal; after adjusting the malting process by reducing the steeping temperature or reducing the fresh air volume and increasing the return air volume during the malting process, the gene expression level is re-analyzed and the principal component score is re-calculated until the ABS is less than 0.5.

11. A application of the malt PYF regulation method based on gene expression during the malting process according to any one of claims 1-10 in improving the PYF performance of malt.

## Patentansprüche

1. Verfahren zur Regulierung der vorzeitigen Hefeausflockung (PYF) in Malz auf der Grundlage der Genexpression während eines Mälzverfahrens, **dadurch gekennzeichnet, dass** es die folgenden Schritte umfasst:
Analysieren der Unterschiede im Genexpressionsniveau zwischen Standardmalz und PYF-Malz im Mälzverfahren und Auswahl von Tracking-Genen in Kombination mit dem Massenproduktionsverfahren, wobei die Tracking-Gene AP1, WRKY70, OSM1, GA2ox3, Thioredoxin, Serpin, RPP13, RbohE, RbohB2, A2, IAA16, BGU17 und WRKY5 sind;
nach dem Entwickeln relevanter Primer gemäß den Sequenzen der Tracking-Gene, dann in der Sequenz, Extrahieren der Gesamt-RNA, Herstellen einer cDNA-Matrize durch reverse Transkriptionsreaktion, Durchführen einer Multiplex-PCR-Reaktion für die 13 Tracking-Gene, Durchführen einer Elektrophorese und Analysieren des Genexpressionsniveaus der Produktfragmente für das zu testende Malz bzw. das Standardmalz; und Vorhersagen und Verbessern der PYF-Leistung des zu testenden Malzes gemäß den folgenden Kriterien:
Berechnen der Hauptkomponentenscores des Standardmalzes und des zu testenden Malzes auf der Grundlage der Genexpressionsniveaus der 13 Tracking-Gene, um einen absoluten Wert der Differenz zwischen den Scores (ABS) zu erhalten; wobei,
wenn der ABS kleiner als 0,5 ist, ein PYF-Wert des zu testenden Malzes ≥ 90 % beträgt, die PYF-Leistung des zu testenden Malzes als normal vorhergesagt wird und keine Notwendigkeit besteht, das Mälzverfahren anzupassen;
wenn der ABS größer als 0,5 und kleiner als 5,0 oder größer als 5,0 ist, die PYF-Leistung des zu testenden Malzes als abnormal vorhergesagt wird und die Einweichtemperatur gesenkt oder das Frischluftvolumen reduziert werden muss und das Rückluftvolumen während des Mälzverfahrens erhöht werden muss, wobei sich das Standardmalz auf ein Malz mit einem PYF-Wert von 100 % bezieht.

2. Verfahren zur PYF-Regulierung in Malz nach Anspruch 1, wobei die Tracking-Gene durch das folgende Verfahren gescreent werden:
Durchführen einer Transkriptomanalyse der Unterschiede im Genexpressionsniveau zwischen dem Standardmalz und dem PYF-Malz in einem Keimungsverfahren des Mälzverfahrens, und Auswahl mehrerer Gene mit größeren Unterschieden im Expressionsniveau als die Kandidatengene;
Tracking des Massenproduktionsverfahrens des Standardmalzes und des PYF-Malzes, und weitere Auswahl von 13 Kandidatengenen mit den offensichtlichsten Veränderungstrends aus den Kandidatengenen als Tracking-Gene.

3. Verfahren zur PYF-Regulierung in Malz nach Anspruch 2, wobei ein Screening-Kriterium für die Tracking-Gene lautet: eine Genexpressionsänderungsamplitude ist größer als oder gleich 2,0 oder kleiner als 0,5; wobei die Genexpressionsänderungsamplitude ein Verhältnis des Genexpressionsniveaus des PYF-Malzes zum Genexpressionsniveau des Standardmalzes ist.

4. Verfahren zur PYF-Regulierung in Malz nach einem der Ansprüche 1 bis 3, wobei die relevanten Primer, die gemäß den Sequenzen der Tracking-Gene entwickelt wurden, einen Primersatz für die reverse Transkriptionsreaktion und einen Primersatz für die Multiplex-PCR-Reaktion umfassen, die für die reverse Transkriptionsreaktion zur Herstellung der cDNA-Matrize bzw. für die Multiplex-PCR-Reaktionen verwendet werden.

5. Verfahren zur PYF-Regulierung in Malz nach Anspruch 4, wobei der Primersatz für die reverse Transkriptionsreaktion für die 13 Tracking-Gene AP1, WRKY70, OSM1, GA2ox3, Thioredoxin, Serpin, RPP13, RbohE, RbohB2, A2, IAA16, BGU17 und WRKY5 entwickelt ist, um 13 Primer zu erhalten, wie dargestellt in SEQ ID NO.2, SEQ ID NO.4, SEQ ID NO.6, SEQ ID NO.8, SEQ ID NO.10, SEQ ID NO.12, SEQ ID NO.14, SEQ ID NO.16, SEQ ID NO.18, SEQ ID NO.20, SEQ ID NO.22, SEQ ID NO.24 bzw. SEQ ID NO.26.

6. Verfahren zur PYF-Regulierung in Malz nach Anspruch 4, wobei der Primersatz für die Multiplex-PCR-Reaktion für die 13 Tracking-Gene AP1, WRKY70, OSM1, GA2ox3, Thioredoxin, Serpin, RPP13, RbohE, RbohB2, A2, IAA16, BGU17 und WRKY5 entwickelt ist, um 13 Primer zu erhalten, wie dargestellt in SEQ ID NO.1, SEQ ID NO.3, SEQ ID NO.5, SEQ ID NO.7, SEQ ID NO.9, SEQ ID NO.11, SEQ ID NO.13, SEQ ID NO.15, SEQ ID NO.17, SEQ ID NO.19, SEQ ID NO.21, SEQ ID NO.23 bzw. SEQ ID NO.25.

7. Verfahren zur PYF-Regulierung in Malz nach einem der Ansprüche 1 bis 3, 5 und 6,
wobei die PYF-Leistung des zu testenden Malzes anhand des folgenden Vorhersagekriteriums vorhergesagt wird:
die Hauptkomponentenscores des Standardmalzes und des zu testenden Malzes werden auf der Grundlage der Genexpressionsniveaus berechnet, und der absolute Wert ABS der Differenz zwischen den Scores wird erhalten; wobei
wenn der ABS kleiner als 0,5 ist, der PYF-Wert des zu testenden Malzes ≥ 90 % beträgt und die PYF-Leistung des zu testenden Malzes als normal vorhergesagt wird;
wenn der ABS größer als 0,5 und kleiner als 5,0 ist, der PYF-Wert des zu testenden Malzes 50-90 % beträgt, die PYF-Leistung des zu testenden Malzes als abnormal vorhergesagt wird und der PYF-Wert erhöht werden muss;
wenn der ABS größer als 5,0 ist, der PYF-Wert des zu testenden Malzes < 50 % beträgt, die PYF-Leistung des zu testenden Malzes als abnormal vorhergesagt wird und der PYF-Wert erhöht werden muss.

8. Verfahren zur PYF-Regulierung in Malz nach Anspruch 7, wobei das Vorhersagekriterium für die PYF-Leistung des zu testenden Malzes durch das folgende Verfahren erhalten wird: Analysieren der Genexpressionsniveaus von Tracking-Genen verschiedener PYF-Malze, um Hauptkomponentenscores der verschiedenen PYF-Malze zu erhalten; Vergleichen des Hauptkomponentenscores jedes der verschiedenen PYF-Malze mit dem Hauptkomponentenscore des Standardmalzes, wodurch das Vorhersagekriterium festgelegt wird.

9. Verfahren zur PYF-Regulierung in Malz nach Anspruch 8, wobei die verschiedenen PYF-Malze PYF-90 %-Malz, PYF-50 %-Malz und PYF-30 %-Malz umfassen; wobei ein absoluter Wert der Differenz zwischen einem Hauptkomponentenscore des PYF-90 %-Malzes und dem Hauptkomponentenscore des Standardmalzes kleiner als 0,5 ist, der absolute Wert der Differenz zwischen einem Hauptkomponentenscore des PYF-50 %-Malzes und dem Hauptkomponentenscore des Standardmalzes größer als 0,5 und kleiner als 5,0 ist, und der absolute Wert der Differenz zwischen einem Hauptkomponentenscore des PYF-30 %-Malzes und dem Hauptkomponentenscore des Standardmalzes größer als 5,0 ist.

10. Verfahren zur PYF-Regulierung in Malz nach einem der Ansprüche 1 bis 3, 5 und 6, wobei, wenn der ABS größer als 0,5 und kleiner als 5,0 oder größer als 5,0 ist, die PYF-Leistung des zu testenden Malzes als abnormal vorhergesagt wird; nach Anpassung des Mälzverfahrens durch Verringerung der Einweichungstemperatur oder Verringerung des Frischluftvolumens und Erhöhung des Rückluftvolumens während des Mälzverfahrens das Genexpressionsniveau erneut analysiert und der Hauptkomponentenscore neu berechnet wird, bis der ABS unter 0,5 liegt.

11. Anwendung des Verfahrens zur PYF-Regulierung in Malz auf der Grundlage der Genexpression während des Mälzverfahrens nach einem der Ansprüche 1-10 zur Verbesserung der PYF-Leistung von Malz.

## Revendications

1. Procédé de régulation de la floculation précoce de la levure (PYF, premature yeast flocculation) du malt reposant sur l'expression génique au cours d'un processus de maltage, **caractérisé en ce qu'**il comprend les étapes suivantes :
l'analyse des différences de niveaux d'expression génique entre un malt standard et un malt à PYF dans le processus de maltage, et la sélection de gènes de suivi en combinaison avec un processus de production en masse, les gènes de suivi étant AP1, WRKY70, OSM1, Ga2ox3, thiorédoxine, serpine, RPP13, RbohE, RbohB2, A2, IAA16, BGU17 et WRKY5 ;
ensuite, après la conception d'amorces pertinentes selon les séquences des gènes de suivi, la réalisation successive de : l'extraction de l'ARN total, la préparation d'un modèle d'ADNc par réaction de transcription inverse, la réalisation d'une réaction PCR multiplexe pour lesdits 13 gènes de suivi, la réalisation d'une électrophorèse et l'analyse du niveau d'expression génique de fragments de produit relatifs au malt à tester et au malt standard, et la prédiction et l'amélioration des performances de PYF du malt à tester, selon les critères suivants :
le calcul de scores des composants principaux concernant le malt standard et le malt à tester compte tenu des niveaux d'expression génique desdits 13 gènes de suivi, pour obtenir une valeur absolue de la différence entre les scores (ABS) ; étant entendu que lorsque l'ABS est inférieure à 0,5, la valeur PYF du malt à tester est ≥ 90 %, les performances de PYF du malt à tester sont prédites comme étant normales, et il n'est pas nécessaire d'ajuster le processus de maltage ;
lorsque l'ABS est supérieure à 0,5 et inférieure à 5,0, ou supérieure 5,0, les performances de PYF du malt à tester sont prédites comme étant anormales et la température de trempage doit être réduite ou le volume d'air frais doit être réduit et le volume d'air de retour doit être augmenté pendant le processus de maltage, le malt dit standard désignant un malt avec une valeur PYF de 100 %.

2. Procédé de régulation de la PYF du malt selon la revendication 1, dans lequel les gènes de suivi sont examinés par le procédé suivant :
la conduite d'une analyse transcriptomique des différences de niveau d'expression génique entre le malt standard et le malt à PYF dans un processus de germination du processus de maltage, et la sélection, comme gènes candidats, de multiples gènes présentant des différences de niveau d'expression plus importantes ;
le suivi du processus de production en masse du malt standard et du malt à PYF, et en outre la sélection, comme gènes de suivi, de 13 gènes candidats présentant les tendances de changement les plus évidentes parmi les gènes candidats.

3. Procédé de régulation de la PYF du malt selon la revendication 2, dans lequel un critère d'examen pour les gènes de suivi est le suivant : l'amplitude de changement de l'expression génique est supérieure ou égale à 2,0 ou inférieure à 0,5, l'amplitude de changement de l'expression génique étant le rapport entre le niveau d'expression génique du malt à PYF et le niveau d'expression génique du malt standard.

4. Procédé de régulation de la PYF du malt selon l'une quelconque des revendications 1 à 3, dans lequel les amorces pertinentes conçues selon les séquences des gènes de suivi comprennent un ensemble d'amorces de réaction de transcription inverse et un ensemble d'amorces de réaction de PCR multiplexe utilisés respectivement pour la réaction de transcription inverse permettant de préparer le modèle d'ADNc et pour les réactions de PCR multiplexe.

5. Procédé de régulation de la PYF du malt selon la revendication 4, dans lequel l'ensemble d'amorces de réaction de transcription inverse est conçu pour les 13 gènes de suivi AP1, WRKY70, OSM1, Ga2ox3, thiorédoxine, serpine, RPP13, RbohE, RbohB2, A2, IAA16, BGU17 et WRKY5 pour obtenir 13 amorces respectivement indiquées dans les SEQ ID N° : 2, SEQ ID N° :4, SEQ ID N° : 6, SEQ ID N° : 8, SEQ ID N° : 10, SEQ ID N° : 12, SEQ ID N° : 14, SEQ ID N° : 16, SEQ ID N° : 18, SEQ ID N° : 20, SEQ ID N° : 22, SEQ ID N° : 24 et SEQ ID N° : 26.

6. Procédé de régulation de la PYF du malt selon la revendication 4, dans lequel l'ensemble d'amorces de réaction de PCR multiplexe est conçu pour les 13 gènes de suivi AP1, WRKY70, OSM1, Ga2ox3, thiorédoxine, serpine, RPP13, RbohE, RbohB2, A2, IAA16, BGU17 et WRKY5 pour obtenir 13 amorces respectivement indiquées dans les SEQ ID N° : 1, SEQ ID N° : 3, SEQ ID N° : 5, SEQ ID N° : 7, SEQ ID N° : 9, SEQ ID N° : 11, SEQ ID N° : 13, SEQ ID N° : 15, SEQ ID N° : 17, SEQ ID N° : 19, SEQ ID N° : 21, SEQ ID N° : 23 et SEQ ID N° : 25.

7. Procédé de régulation de la PYF du malt selon l'une quelconque des revendications 1 à 3, 5 et 6, dans lequel les performances de PYF du malt à tester sont prédites par le critère de prédiction suivant :
les scores des composants principaux du malt standard et du malt à tester sont calculés compte tenu des niveaux d'expression génique, et la valeur absolue ABS de la différence entre les scores est obtenue ; étant entendu que
lorsque l'ABS est inférieure à 0,5, la valeur PYF du malt à tester est ≥ 90 % et les performances de PYF du malt à tester sont prédites comme étant normales ;
lorsque l'ABS est supérieure à 0,5 et inférieure à 5,0, la valeur PYF du malt à tester va de 50 % à 90 %, les performances de PYF du malt à tester sont prédites comme étant anormales et la valeur PYF doit être augmentée ;
lorsque l'ABS est supérieure à 5,0, la valeur PYF du malt à tester est < 50 %, les performances de PYF du malt à tester sont prédites comme étant anormales et la valeur PYF doit être augmentée.

8. Procédé de régulation de la PYF du malt selon la revendication 7, dans lequel le critère de prédiction des performances de PYF du malt à tester est obtenu par le procédé suivant : l'analyse des niveaux d'expression génique de différents malts PYF pour obtenir des scores des composants principaux des différents malts PYF ; la comparaison du score des composants principaux de chacun des différents malts PYF au score des composants principaux du malt standard, établissant ainsi le critère de prédiction.

9. Procédé de régulation de la PYF du malt selon la revendication 8, dans lequel les différents malts PYF comprennent un malt à PYF de 90 %, un malt à PYF de 50 % et un malt à PYF de 30 %, la valeur absolue de la différence entre le score de composants principaux du malt à PYF de 90 % et le score de composants principaux du malt standard étant inférieure à 0,5, la valeur absolue de la différence entre le score de composants principaux du malt à PYF de 50 % et le score de composants principaux du malt standard étant supérieure à 0,5 et inférieure à 5,0, et la valeur absolue de la différence entre le score de composants principaux du malt à PYF de 30 % et le score de composants principaux du malt standard étant supérieure à 5,0.

10. Procédé de régulation de la PYF du malt selon l'une quelconque des revendications 1 à 3, 5 et 6, dans lequel, lorsque l'ABS est supérieure à 0,5 et inférieure à 5,0, ou supérieure à 5,0, les performances de PYF du malt à tester sont prédites comme étant anormales ; après ajustement du processus de maltage par réduction de la température de trempage ou par réduction du volume d'air frais et augmentation du volume d'air de retour au cours du processus de maltage, le niveau d'expression génique est réanalysé et le score des composants principaux est recalculé jusqu'à ce que l'ABS soit inférieure à 0,5.

11. Application du procédé de régulation de la PYF du malt reposant sur l'expression génique au cours du processus de maltage selon l'une quelconque des revendications 1 à 10 dans l'amélioration des performances de PYF du malt.
